# EUROPEAN PATENT SPECIFICATION

(11) **EP 1 283 019 B1**
(45) Date of publication and mention of the grant of the patent: **18.10.2006**
(21) Application number: 02255494.3
(22) Date of filing: 06.08.2002
(51) Int. Cl.: A47K 7/02, A61K 8/02, A61Q 5/00, A61Q 19/00

(54) **Textured article**
Texturierter Artikel
Article texturé

(30) Priority: 07.08.2001 US 923552
(43) Date of publication of application: 12.02.2003
(73) Proprietor: JOHNSON & JOHNSON CONSUMER COMPANIES, INC., Skillman, New Jersey 08558 (US)
(72) Inventor: McMeekin, Linda J., Bound Brook, NJ 08805 (US); Luizzi, Joseph, Newtown, PA 18940 (US)
(74) Representative: Mercer, Christopher Paul

(56) References cited:
- WO-A-99/13861
- WO-A-99/25318

## Description

### BACKGROUND OF THE INVENTION

### 1. Field Of The Invention

The present invention relates to a textured article. The article contains raised elements on at least one surface. The raised elements provide gentle massaging, cleansing, scrubbing and/or exfoliating action for skin, hair or surface treatment. The article may contain an active that is intended to be applied to, or interact with, the surface of the skin or hair. The substrate for the article may be a woven or knit fabric, a nonwoven, a laminate containing a fabric and a polymeric film, a flocked fabric, and combinations thereof.

### 2. Description Of The Prior Art

In recent years, many articles have been developed to aid in wiping various substrates. One example is the development of baby wipes, which are used to wipe the baby clean during diaper changes. Baby wipes typically are soft and are loaded with a cleanser and moisturizer. Baby wipes are not known for exfoliating properties. As used herein, exfoliating means removing dead skin from the surface of the skin.

Another type of wipe that has been developed is the hand wipe. Hand wipes are used to clean the hands when the use of a sink is inconvenient. These wipes typically are not as soft as baby wipes. Hand wipes frequently contain both cleansers and antibacterial agents. Hand wipes are not known to have exfoliating properties.

Wipes have also been developed for cleaning the face. These wipes are typically very soft and contain cleansers, moisturizers, and anti-acne agents. Most face wipes are not known for exfoliating properties. The BUFF-PUFF® pad is sold commercially for exfoliating and cleansing the face. The pad is made from a spun-bond polymer and has a very rough texture. Although the pad is effective at cleansing and exfoliating the skin, some consumers find the pad to be too rough. Therefore, there is a need for a wipe that cleans and exfoliates the skin without being too rough on the skin.

It is known to selectively place rubbery materials on a cloth surface to improve friction and reduce slip. For example, TOTES® slipper socks are made of a fabric that would be slippery on floor surfaces. To overcome this problem, the fabric is treated to place raised rubbery elements in the form of dots on the surface of the fabric that contacts the floor.

Another example of selectively placing materials on a cloth substrate is disclosed in United States Patent No. 5,538,732. The patent teaches selectively placing dots of water soluble active ingredients on wipes. In use, the wipes are wetted, then applied to the skin to deliver the active ingredients. The dots are not raised and do not exfoliate or massage the skin.

WO99/25318 discloses a personal cleansing wipe comprising, *inter alia,* "a single layer, nonwoven substrate formed from hydroentangled fibers", a three-dimensional pattern, and an aqueous liquid cleansing composition "comprising an effective amount of a cleansing surfactant", said aqueous liquid cleansing composition being coated onto or impregnated into the substrate in particular proportions.

WO99/13861 discloses a "disposable, single use personal care cleansing and conditioning article" comprising, *inter alia,* a water insoluble substrate "wherein at least a first portion of said substrate is wet extensible and at least a second portion of said substrate is less wet extensible than said first portion" and at least one lathering surfactant added onto or impregnated into the substrate, wherein said article is "substantially dry prior to use".

Despite the disclosure of the prior art, there remains a need for a wipe that cleans and exfoliates the skin without being too rough on the skin.

### SUMMARY OF THE INVENTION

The present invention provides a textured article comprising:
a substrate having at least two surfaces; and
raised elements on at least one surface of the substrate,
wherein the article is useful for providing skin or hair care benefits and wherein the raised elements are formed from hot melt coatings comprising an olefin polymer, a block copolymer or a combination thereof.

### Detailed Description of Preferred Embodiments

The article of the invention includes a substrate. Suitable substrates are known in the art of wipes and include, but are not limited to, a woven fabric, a knit fabric, a nonwoven fabric, a laminate of a fabric and a polymeric film, such as a polyolefin film, a flocked fabric, and combinations thereof. Methods of making woven and knit cloths are not a part of this invention and, being well known in the art, are not described in detail herein. One type of nonwoven cloth substrate utilized in the present invention is made by air- or water-laying processes in which the fibers or filaments are first cut to desired lengths from long strands, passed into a water or air stream, and then deposited onto a screen through which the fiber-laden air or water is passed. The deposited fibers or filaments are then adhesively bonded together, and otherwise treated as desired to form the woven, nonwoven, or cellulose cloth.

In another embodiment, the substrate utilized in the present invention may be a thermal bonded nonwoven cloth (whether or not resin-containing) which can be made of polyesters, polyamides, polyolefins, or other thermoplastic fibers which can be spun bonded, i.e., the fibers are spun out onto a flat surface and bonded (melted) together by heat or chemical reactions.

When nonwoven substrates are utilized, the nonwoven cloth substrates are generally adhesively bonded fibers or filamentous products having a web or carded fiber structure (when the fiber strength is suitable to allow carding) or comprising fibrous mats in which the fibers or filaments are distributed haphazardly or in random array (i.e., an array of fibers in a carded web where partial orientation of the fibers is frequently present, as well as a completely haphazard distributional orientation), or substantially aligned. The fibers or filaments can be natural (e.g., wool, silk, jute, hemp, cotton, linen, sisal, or ramie) or synthetic (e.g., rayon, cellulose ester, polyvinyl derivatives, polyolefins, such as polyethylene and polypropylene, polyamides, such as nylon 6, nylon 6,6, or polyesters, such as polyethylene terephthalate and polybutylene terephthalate), or combinations thereof. These nonwoven materials are generally described in the INDA "NONWOVEN FABRICS HANDBOOK", (1999), for nonwoven substrates and their methods of manufacture. The basis weight of the substrate may vary, but generally ranges from about 20 grams per square meter to about 500 grams per square meter, for example from about 50 grams per square meter to about 150 grams per square meter.

The substrate has at least two surfaces, generally a top surface and a bottom surface. The article of the invention is useful for exfoliating skin, therefore the article contains raised elements on at least one surface of the substrate. The raised elements may be discrete.

The raised elements are formed from hot melt coatings comprising an olefin polymer, a block copolymer or a combination thereof. The raised elements are made of a material suitable for providing abrasive or massaging properties. Suitable materials may comprise natural rubber, synthetic rubber, polyolefins, such as polyethylene and polypropylene, ethylene vinyl acetate, and thermoplastic elastomers. Colorants or pigments may be combined with the coating materials.

Suitable hot melt coatings for generating raised elements include HL-7471 W from H.B. Fuller Co., St. Paul, MN, and REXTAC amorphous polyolefins, available through Huntsman Chemical. For example, hot melt coatings containing from about 15% to about 100% olefin polymer or a block copolymer, from about 0% to about 60% tackifying resin, and from about 0% to about 50% wax may be useful. Suitable olefin polymers include polymers:
a) wherein the olefin polymer is a homopolymer of ethylene, propylene, n-butene, butylene or isobutylene, with a melt flow index from 0.5 to 2500, such as Ateva^{™} polymers from AT plastics; Escorene® and Vistanex® polymers from Exxon Chemical, Duraflex® polymers from Shell Chemical, Epolene® polymers from Eastman Chemical, and Vestoplast® polymers from Creanova;
b) wherein the olefin polymer is a copolymer of ethylene and a co-monomer, such as vinyl acetate, acrylic acid, methacrylic acid, ethyl acrylate, methyl acrylate, n-butyl acrylate vinyl silane or maleic anhydride, such as Ateva^{™} polymers from AT plastics, Elvax® polymers from DuPont, Escorene® and Optema® polymers from Exxon Chemical, and Primacor® polymers from Dow Chemical; and
c) wherein the olefin polymer is a terpolymer of ethylene and co-monomers, such as vinyl acetate, acrylic acid, methacrylic acid, ethyl acrylate, methyl acrylate, n-butyl acrylate vinyl silane or maleic anhydride, such as Ateva^{™} polymers from AT plastics, Nucrel® polymers from DuPont, and Escor® polymers from Exxon Chemical.

Suitable block copolymers include block copolymers having a linear or a radial structure such that the structure (A―B)ₓ where A is consists essentially of a polyvinylarene block, and B consists essentially of poly(monoalkenyl) block, and x denotes the number of polymeric arms, where x is greater than or equal to one are also useful. Block B may be selected from conjugated diene elastomers such as polybutadiene or polyisoprene and hydrogenated elastomers such as ethylene-butylene or ethylene- propylene. Suitable examples of these types of polymers include Kraton® elastomers from Shell Chemical Company, Vector® elastomers from Dexco, Solprene® elastomers from Enichem Elastomers and Stereon® from elastomers Firestone Tire & Rubber Co. When the hot melt coatings contain block copolymers, it is preferable for the coating to contain from about 15% to about 50% block copolymer.

Suitable tackifying resins include any compatible resin or mixture thereof selected from the group consisting of a) natural and modified rosins; b) glycerol and pentaerythritol esters of natural and modified rosins; c) polyterpene resins; d) copolymers and terpolymers of natural terpenes; e) phenolic modified terpene resins and the hydrogenated derivatives thereof; f) aliphatic petroleum resins and the hydrogenated derivatives thereof; g) aromatic petroleum resin and the hydrogenated derivatives thereof; and h) aliphatic/aromatic petroleum resins and the hydrogenated derivatives thereof, such as Foral® resin, Staybelite® resin, Poly pale® resin, Permalyn® resin, Pentalyn® resin, Adtac® resin, Piccopale® resin, Piccotac® resin, Hercotac® resin, Regalrez® resin, and Piccolyte® resin from Hercules, Escorez® resin from Exxon Chemical, Wingtack® resin from Goodyear Tire & Rubber Co., Arkon® resin from Arakawa Chemicals, Zonatac® resin, Zonarez® resin and Zonester® resin from Arizona Chemical and Nevtac® resin from Neville Chemical Company.

Suitable waxes include, but are not limited to, paraffins, Fischer-tropsh, and microcrystalline waxes, and combinations thereof. Suitable microcrystalline waxes include, but are not limited to, BE SQUARE 175 microwax, available from Bareco Division, Petrolite Corporation, and M-5165 from Moore & Munger, Shelton, CT.

Suitable polyethylene waxes include, but are not limited to, H-101 from Exxon Chemical, Houston, TX. Suitable Fischer-Tropsch waxes include, but are not limited to, Paraflint Wax from Schumann Sasol, Hamburg, Germany.

The raised elements may be applied onto the substrate by any means known in the art, such as control coating, control fiberization, pattern coating, gravure coating, rotary screen printing, and spray coating. Equipment for coating the substrates is commercially available. One example is the DYNAFIBER, available through Nordson Company. Another example is the ITW, available through Omega Company. When applying raised elements through a melt process, the time it takes to cool the applied coating affects the height of the raised elements. If the coating is not cooled quickly enough, the coating may penetrate the substrate to the extent that no raised element is formed. To overcome this problem, an air knife that utilizes air, which may be chilled, may be utilized to quickly cool the applied coating and prevent tailing. The angle of contact between the air and the applied coating may also affect the height of the raised elements. The air typically contacts the coating at an angle of from about 10° to about 80°.

The raised elements may be of any shape including, but not limited to, lines, waves, interconnected patterns, circular dots, hexagons, hearts, diamonds, rectangles, stars, triangles and the like. The density, height, and diameter of the raised elements may vary depending on the massaging or exfoliating properties desired. Generally, the raised elements may occupy anywhere from about 1 percent to about 99 percent, for example from about 10 percent to about 75 percent, or from about 20 percent to about 50 percent of the surface of at least one side of the substrate. The height of the raised elements, as measured from the surface of the substrate, should be sufficient to provide exfoliating or massaging properties. Generally, the height is at least 0.01 mm above the surface of the substrate.

When the raised elements are discrete, the elements have a diameter sufficient to provide exfoliating or massaging properties. Generally, the diameter of the discrete raised elements may range from about 0.5 mm to about 3 mm, for example about 1 mm to about 2 mm.

The articles of the present invention provide a skin care benefit or a hair care benefit. As used herein, skin care benefit and hair care benefit means massaging, cleansing, scrubbing and/or exfoliating the skin.

The articles of the invention are preferably combined, e.g., coated or impregnated, with cleansers, skin and/or hair care actives, moisturizers, and the like. The articles may be loaded with appropriate skin and/or hair care compositions and packaged as wet wipes. The skin care or hair care compositions may be loaded onto the wipes by dipping the wipes in the skin care composition, spraying the skin care composition onto the wipes, and other means known in the art.

Alternatively, the wet wipe may be dried through the use of heating equipment or vacuum driers to provide dry wipes. Alternatively, a cleansing or skin care formulation may be applied in the form of a concentrate to the substrate to provide dry textured articles. The dry textured articles or wipes are sold dry, then the consumer wets the wipe with water when ready for use.

Several examples are set forth below to further illustrate the nature of the invention and the manner of carrying it out. However, the invention should not be considered as being limited to the details thereof.

### Example 1:

A textured article was prepared on a Kramer Coater utilizing 80 gsm Jacob Holm (100% polyethylene terephthalate spunlace nonwoven) as the substrate and Fuller HL-7471W (with titanium dioxide) as the discreet raised elements. The HL-7471W was melted at 350°F and pattern applied to the substrate using a random dot screen pattern number 50129 (Rothtec Engraving Corporation) at a coat weight of 34.4 g/m². The dot screen pattern had 146,500 dots per square meter. The circular dots had an average diameter of 1.28 mm, a height of 0.08 mm, and were spaced apart approximately 3.57 mm.

### Example 2:

A second textured article was prepared on a May Coater utilizing Orlandi 55 gsm (70% polyester/30% Rayon spunlace nonwoven) as the substrate and Fuller HL-7471W (with titanium dioxide) as the discreet raised elements. The HL-7471W was melted at 325°F and pattern applied to the substrate at a coat weight of 38.06 g/yd² using a Rothtec random dot screen. The dot screen pattern had 146,500 dots per square meter. The circular dots had an average diameter of 1.36 mm, a height of 0.04 mm, and were spaced apart approximately 3.29 mm.

## Claims

1. A textured article comprising:
a substrate having at least two surfaces; and
raised elements on at least one surface of the substrate,
wherein the article is useful for providing skin or hair care benefits and wherein the raised elements are formed from hot melt coatings comprising an olefin polymer, a block copolymer or a combination thereof

2. The article of claim 1 wherein the substrate is a woven fabric, a knit fabric, a nonwoven fabric, a laminate of a fabric and a polymeric film, a flocked fabric or a combination thereof.

3. The article of claim 1 or claim 2 wherein the hot melt coating comprises from 15% to 100% olefin polymer or a block copolymer, from 0% to 60% tackifying resin and from 0% to 50% wax.

4. The article of any one of claims 1 to 3 wherein the raised elements are discrete and are in a shape of circular dots, hexagons, hearts, diamonds, rectangles, stars or triangles.

5. The article of claim 4 wherein the discrete raised elements cover from 10 percent to 90 percent of the surface of at least one side of the substrate.

6. The article of claim 4 or claim 5 wherein the discrete raised elements have a diameter ranging from 0.5 mm to 3 mm.

7. The article of any one of claims 4 to 6 wherein the discrete raised elements have a height greater than about 0.01 mm as measured from the surface of the substrate.

8. The article of any one of claims 1 to 7 wherein the substrate has a basis weight ranging from 20 grams per meter to 500 grams per square meter.

9. The article of any one of claims 1 to 8 further comprising a skin or hair care composition.

10. The article of any one of claims 1 to 9 in the form of a dry or wet wipe.

11. A method for producing an exfoliating wipe as described in claim 1, said method comprising:
providing a substrate comprising a woven fabric, a knit fabric, a nonwoven fabric, a laminate of a fabric and a polymeric film, a flocked fabric or a combination thereof; and
pattern coating raised elements onto the substrate utilizing a hot melt coating of an olefin polymer, a block copolymer or a combination thereof.

12. The method of claim 11 wherein the pattern coating is applied by hot melt rotary screen coating or hot melt gravure coating.

13. The article of any one of claims 1 to 9 for use in cleansing the skin or hair by topical application or for use in cleansing, exfoliating or massaging the skin by topical application.

14. The article of any one of claims 4 to 7 wherein the substrate has a basis weight ranging from 50 grams per meter to 150 grams per square meter.

## Patentansprüche

1. Texturierter Artikel, umfassend:
ein Substrat mit zumindest zwei Oberflächen; und
erhabene Elemente auf zumindest einer Oberfläche des Substrats,
**dadurch gekennzeichnet, daß** der Artikel geeignet ist für die Bereitstellung von Haut- oder Haarpflegevorzügen, wobei die erhabenen Elemente aus Hot-Melt-Beschichtungen gebildet sind, die ein Olefinpolymer, ein Block-Copolymer oder eine Kombination derselben umfassen.

2. Artikel nach Anspruch 1, **dadurch gekennzeichnet, daß** das Substrat ein Webstoff, ein Strickstoff, ein Faservliesstoff, ein Laminat eines Gewebes und eines polymeren Films, ein Flockgewebe oder eine Kombination derselben ist.

3. Artikel nach Anspruch 1 oder Anspruch 2, **dadurch gekennzeichnet, daß** die Hot-Melt-Beschichtung 15 % bis 100 % Olefinpolymer oder eines Block-Copolymers, 0 % bis 60 % klebrig machendes Harz und 0 % bis 50 % Wachs umfaßt.

4. Artikel nach einem der Ansprüche 1 bis 3, **dadurch gekennzeichnet, daß** die erhabenen Elemente einzeln stehend sind und die Form von kreisförmigen Tupfen, Sechsecken, Herzen, Diamanten, Rechtecken, Sternen oder Dreiecken haben.

5. Artikel nach Anspruch 4, **dadurch gekennzeichnet, daß** die einzeln stehenden erhabenen Elemente 10 Prozent bis 90 Prozent der Oberfläche zumindest einer Seite des Substrats bedecken.

6. Artikel nach Anspruch 4 oder Anspruch 5, **dadurch gekennzeichnet, daß** die einzeln stehenden erhabenen Elemente einen Durchmesser im Bereich von 0,5 mm bis 3 mm aufweisen.

7. Artikel nach einem der Ansprüche 4 bis 6, **dadurch gekennzeichnet, daß** die einzeln stehenden erhabenen Elemente eine Höhe von mehr als zirka 0,01 mm haben, gemessen von der Oberfläche des Substrats aus.

8. Artikel nach einem der Ansprüche 1 bis 7, **dadurch gekennzeichnet, daß** das Substrat ein Basisgewicht von 20 Gramm pro Quadratmeter bis 500 Gramm pro Quadratmeter hat.

9. Artikel nach einem der Ansprüche 1 bis 8, der weiterhin eine Haut- oder Haarpflege-Zusammensetzung umfaßt.

10. Artikel nach einem der Ansprüche 1 bis 9 in Form eines Trocken- oder Feuchttuches.

11. Verfahren für die Herstellung eines exfolierenden Tuches, wie in Anspruch 1 beschrieben, wobei das Verfahren umfaßt:
Bereitstellung eines Substrats, welches einen Webstoff, einen Strickstoff, einen Faservliesstoff, ein Laminat eines Gewebes und eines polymeren Films, ein Flockgewebe oder eine Kombination derselben umfaßt; und
Musterbeschichtung der erhabenen Elemente auf das Substrat unter Verwendung einer Hot-Melt-Beschichtung aus einem Olefinpolymer, einem Block-Copolymer oder einer Kombination derselben.

12. Verfahren nach Anspruch 11, **dadurch gekennzeichnet, daß** die Musterbeschichtung mit Hilfe von Hot-Melt-Rotationssiebbeschichtung oder Hot-Melt-Gravurbeschichtung aufgebracht wird.

13. Artikel nach einem der Ansprüche 1 bis 9 für die Verwendung bei der Reinigung der Haut oder des Haares durch äußerliche Anwendung oder für die Verwendung bei der Reinigung, beim Exfolieren oder für die Massage der Haut durch äußerliche Anwendung.

14. Artikel nach einem der Ansprüche 4 bis 7, **dadurch gekennzeichnet, daß** das Substrat ein Basisgewicht von 50 Gramm pro Quadratmeter bis 150 Gramm pro Quadratmeter hat.

## Revendications

1. Article texturé comprenant :
un substrat ayant au moins deux surfaces, et
des éléments élevés sur au moins une surface du substrat,
dans lequel l'élément est utile pour prodiguer des soins dermatologiques ou capillaires, et dans lequel les éléments élevés sont formés à partir de revêtements fondus à chaud comprenant un polymère oléfine, un copolymère en blocs ou un de leurs mélanges.

2. Article selon la revendication 1 dans lequel le substrat est un tissu tissé, un tissu tricoté, un tissu non tissé, un stratifié d'un tissu et d'un film polymère, un tissu floqué ou un de leurs mélanges.

3. Article selon la revendication 1 ou 2 dans lequel le revêtement fondu à chaud comprend de 15% à 100% de polymère oléfine ou un copolymère en blocs, de 0% à 60% de résine collante et de 0% à 50% de cire.

4. Article selon l'une quelconque des revendications 1 à 3 dans lequel les éléments élevés sont discrets et ont la forme de points circulaires, d'hexagones, de coeurs, de diamants, de rectangles, d'étoiles ou de triangles.

5. Article selon la revendication 4 dans lequel les éléments élevés discrets couvrent de 10% à 90% de la surface d'au moins un côté du substrat.

6. Article selon la revendication 4 ou 5 dans lequel les éléments élevés discrets ont un diamètre de 0,5 mm à 3 mm.

7. Article selon l'une quelconque des revendications 4 à 6 dans lequel les éléments élevés discrets ont une hauteur supérieure à environ 0,01 mm mesurée à partir de la surface du substrat.

8. Article selon l'une quelconque des revendications 1 à 7 dans lequel le substrat a une masse de base qui va de 20 g par mètre à 500 g par mètre carré.

9. Article selon l'une quelconque des revendications 1 à 8 comprenant en outre une composition de soin dermatologique ou capillaire.

10. Article selon l'une quelconque des revendications 1 à 9 sous la forme d'une lingette sèche ou humide.

11. Procédé de production d'une lingette exfoliante comme décrite dans la revendication 1, ledit procédé comprenant :
la fourniture d'un substrat comprenant un tissu tissé, un tissu tricoté, un tissu non tissé, un stratifié d'un tissu et d'un film polymère, un tissu floqué ou un de leurs mélanges, et
le revêtement selon un motif d'éléments élevés sur le substrat, en utilisant un revêtement fondu à chaud d'un polymère oléfine, un copolymère en blocs ou un de leurs mélanges.

12. Procédé selon la revendication 11 dans lequel le revêtement selon un motif est appliqué par couchage par bobine ou par couchage par gravure de revêtement fondu à chaud.

13. Article selon l'une quelconque des revendications 1 à 9 à utiliser pour nettoyer la peau ou les cheveux par application locale ou à utiliser pour nettoyer, exfolier ou masser la peau par application locale.

14. Article selon l'une quelconque des revendications 4 à 7 dans lequel le substrat a une masse de base qui va de 50 g par mètre et 150 g par mètre carré.
